Europäisches Patentamt

European Patent Office

Office européen des brevets

(11) Veröffentlichungsnummer: **0 115 001**
**B1**

(12) **EUROPÄISCHE PATENTSCHRIFT**

(45) Veröffentlichungstag der Patentschrift: **15.06.88**

(21) Anmeldenummer: **83112683.4**

(22) Anmeldetag: **16.12.83**

(51) Int. Cl.$^4$: **C 07 H 15/22, A 61 K 31/43,**
**A 61 K 31/70, A 61 K 9/10**

(54) Stabile Aminoglykosid-Penicillin-Injektionsformulierungen.

(30) Priorität: **28.12.82 DE 3248328**
**18.03.83 DE 3309763**

(43) Veröffentlichungstag der Anmeldung:
**08.08.84 Patentblatt 84/32**

(45) Bekanntmachung des Hinweises auf die
Patenterteilung:
**15.06.88 Patentblatt 88/24**

(84) Benannte Vertragsstaaten:
**AT BE CH DE FR GB IT LI NL**

(56) Entgegenhaltungen:
**FR-A-2 186 230**
**FR-A-2 374 031**
**US-A-3 636 194**
**US-A-4 172 138**

**Med. Mo. Pharm., 5 (1982), S. 151**
**Infection 4 (1976) Nr. 2, S. 107-109**
**Am. J. Hosp. Pharm.,38 (1981), S. 1167-1170**
**Physicians Desk Ref. 35 (1981) S. 1930**

(73) Patentinhaber: **BAYER AG**
**Konzernverwaltung RP Patentabteilung**
**D-5090 Leverkusen 1 Bayerwerk (DE)**

(72) Erfinder: **Voege, Herbert, Dr.**
**Martin-Buber-Strasse 41**
**D-5090 Leverkusen 3 (DE)**
Erfinder: **Stadler, Peter, Dr.**
**Am Ideck 8**
**D-5657 Haan (DE)**
Erfinder: **Zeiler, Hans-Joachim, Dr.**
**Elsbeekerstrasse 46**
**D-5604 Neviges (DE)**
Erfinder: **Samaan, Samir, Dr.**
**Claudiusweg 9**
**D-5600 Wuppertal 1 (DE)**
Erfinder: **Metzger, Karl Georg, Dr.**
**Pahlkestrasse 15**
**D-5600 Wuppertal (DE)**

Courier Press, Leamington Spa, England.

**Beschreibung**

Die vorliegende Erfindung betrifft Injektionsformulierungen bestehend aus wenigstens einem zur Injektion geeigneten Öl, wenigstens einem Aminoglykosid und wenigstens einem Penicillin zur Applikation in der Human- und Veterinärmedizin, wobei Formulierungen zur Infusion in Körperhöhlen ausgenommen sind, die Verwendung von zur Injektion geeigneten Ölen zur Herstellung stabiler Aminoglykoside und Penicilline enthaltender Injektionsformulierungen sowie die Herstellung solcher Injektionsformulierungen.

Aus mikrobiologischer Sicht wäre ein Kombinationsprodukt aus Penicillinen und Aminoglykosiden äußerst erwünscht, da dann fast das gesamte gramnegative sowie grampositive Bakterien-Spektrum erfaßt würde. Leider können aber zur Zeit beide Wirkstoffe nur getrennt an verschiedenen Körperstellen injiziert werden, wenn von beiden Wirkstoffen optimale Wirksamkeit erhalten werden soll. Die beiden Injektionslösungen dürfen auch nicht vor Gebrauch in einer Spritze zusmamen verwendet werden (Anwendungsempfehlungen der Aminoglykosid-Lösungs-Hersteller oder I. Carrizosa und D. Kaye, Antimicrobial Agents and Chemotherapy, 13 (3), S. 505—508 (1978)). Der Grund dafür ist in einer chemischen Inkompatibilität zwischen Penicillinen und Aminoglykosiden zu finden, wie sie von J. Herderson et. al., Amer. J. Hosp. Pharm., *38*, 1167 (1981) beschrieben wurde.

Antibiotika aus der Reihe der Aminoglykoside werden seit langem in der Human- und Veterinär-Medizin zur Bekämpfung von bakteriellen Infektionen eingesetzt. Insbesondere in der Veterinär-Medizin besteht bei derartigen Erkrankungen der Wunsch, schon nach nur einmaliger Injektion des Wirkstoffes einen Heilungserfolg zu erreichen. Dies ist zum Beispiel bei Penicillinen durchaus möglich, wenn den tieren eine Suspension schwerlöslicher Penicillin-Salze injiziert wird, deren Wirkstoff erst nach und nach freigegeben wird. Dadurch wird ein wirksamer Penicillin-Blutspiegel über einen längeren Zeitraum erhalten und eine mehrfache Wiederholung der Injektion wird unnötig.

Bei Aminoglykosiden, die üblicherweise als wäßrige Lösung injiziert werden, ist die biologische Halbwertzeit relativ kurz (ca 1 h), so daß bei einer Infektion mehrmals am Tage eine Injektion vorgenommen werden muß.

Es wäre also wünschenswert und für den Tierhalter und den Tierarzt eine große Erleichterung, wenn die Anzahl der Injektionen während der Erkrankung des Tieres reduziert werden könnte.

Injektionsformulierungen von Aminoglykosiden mit diesen Eigenschaften sind bisher nicht bekannt. Wohl sind in anderem Zusammenhang Arzneiformen beschrieben worden, bei denen Aminoglykoside kontinuierlich über einen verlängerten Zeitraum freigesetzt werden. So werden beispielsweise in den US-Patenten 4 188 373 und 4 115 544 Augentropfen geschützt, die Gentamicin verzögert freisetzen. Bekannt sind auch Kunststoff- oder Keramik-Arzneiformen, die in infizierte Knochen implantiert werden und dort das Aminoglykosid kontinuierlich freigeben (siehe z.B. DE—OS 2 815 934, DE—OS 3 005 350, DL 139 942 oder DE—OS 2 807 132).

Auch die Herstellung schwerlöslicher Salze wurde für Aminoglykoside bereits beschrieben. Diese sind jedoch ebenfalls nur für die äußerliche Anwendung gedacht, nicht aber für Injektionsformulierungen. So werden in der DE—OS 2 301 633 wasserunlösliche Antibiotikasalze beansprucht, bei denen aus Gentamicin bzw. Polymyxin als Basen und Penicillin bzw. Cephalosporinen als Säuren das Salz hergestellt wird. Diese Salze haben den Nachteil, daß das Verhältnis Aminoglykosid/Penicillin-Derivat durch das Mol-Verhältnis fixiert ist und nicht dem bakteriologisch sinnvollen Optimum angepaßt werden kann.

Mit einer langsam Wirkstoff freisetzenden Aminoglykosid-Formulierung wäre auch ein Kombinationspräparat mit β-Lactamantibiotika zu verwirklichen, welches von den Nachteilen des oben beschriebenen Penicillin/Gentamicinsalzes frei ist:

Es ist zwar bereits aus DE—OS 2 756 079 bekannt, ein Kombinationspräparat aus Aminoglykosid und Penicillin-Derivaten herzustellen, indem das Penicillin mit Polyvinylpyrrolidon und Lecithin beschichtet wird. Das getrocknete Pulver wird in sterile Glasflaschen abgefüllt und kann vor Gebrauch durch Zugabe einer wäßrigen Aminoglykosid-Lösung zum Kombinationspräparat angeschüttelt werden. Die Herstellung dieses Präparats its jedoch aufwendig und teuer und eine "ready to use" Suspension kann so nicht hergestellt werden.

Aus US—P 4 172 138 und 3 636 194 sind antibiotikahaltige ölige Euterinfusionslösungen bekannt. Solche Lösungen sind jedoch nicht zur z.B. intramuskulären Injektion geeignet.

Überraschenderweise gelingt es erfindungsgemäß erstmalig, Injektionsformulierungen, ausgenommen Formulierungen zur Infusion in Körperhöhlen, herzustellen, die Wirkstoffe aus beiden Wirkstoffklassen enthalten und chemisch stabil sind, so daß sie als Kombinationsprodukt hergestellt und eingesetzt werden können, wenn man die Wirkstoffe in einem injizierbaren Öl suspendiert, in dem sie sich nicht lösen.

Aus der Gruppe der Aminoglykoside sind vor allem Gentamicin, Sisomicin, Etomicin, Amikacin, Bluensomycin, Neomycin, Paromomycin, Lividomycin, Kanamycin, Dibekacin, Nebramycin, Ribostamycin, Butirosin, Kasugamycin, Sagamicin, Apramycin, Verdamicin, Xylostasin, Destomycin, Hygromycin, Seldomycin, Umtamicin, Tobramycin, Spectinomycin und Fortimicin als Formulierungskomponenten geeignet.

Besonders bevorzugt aus dieser Gruppe sind vor allem Gentamycin, Sisomicin, Etomicin, Amikacin, Neomycin, Kanamycin, Tobramycin und Fortimicin.

Aus der Gruppe der Penicilline können alle biosynthetischen oder halbsynthetischen Penicilline

2

eingesetzt werden vor allem jedoch: Benzylpenicillin, Phenoxymethyl-Penicillin, Propicillin, Phenethicillin, Oxacillin, Cloxacillin, Dicloxacillin, Ampicillin, Carbenicillin, Methicillin, Metampicillin, Acidocillin, Amoxycillin, Ticarcillin, Ticcercillin, Talampicillin, Pivampicillin, Epicillin, Ciclacillin, Indamylcarbenicillin. Ebenso Ureido-Penicilline wie z.B. Mezlocillin, Azlocillin und Piperacillin.

Besonders bevorzugt aus dieser Gruppe sind vor allem Benzylpenicillin, Oxacillin, Cloxacillin, Dicloxacillin und Ampicillin. Sowohl die Substanzen aus der Gruppe der Aminoglykoside als auch die Penicilline können in Form ihrer freien Säure bzw. Base oder in Form ihrer Salze eingesetzt werden. Auch Salze, die sich nur wenig in Wasser lösen und daher eine verzögerte Wirkstoffabgabe bewirken, können eingesetzt werden. Bei Penicillinen sind das z.B. die Procainsalze oder Benzathinsalze.

In Wasser schwer lösliche Salze von Aminoglykosidantibiotika sind solche mit nicht antibiotisch wirkenden organischen Carbon- oder Sulfonsäuren oder Halbestern von mehrbasischen anorganischen Säuren mit aliphatischen Alkoholen.

Unter "schwer löslich" im Sinne der vorliegenden Erfindung ist zu verstehen, daß sich bei 20°C 1 bis 1000 mg des Salzes in 1000 ml Wasser lösen, vorzugsweise 1 bis 500, besonders bevorzugt 5 bis 200 mg.

Als Aminoglykosidantibiotika zur Salzbildung sind vorzugsweise die folgenden geeignet:

Gentamicin, Sisomicin, Etomicin, Amikacin, Streptomycin, Bluensomycin, Neomycin, Paromomycin, lividomycin, Kanamycin, Dibekacin, Nebramycin, Ribostamycin, Butirosin, Kasugamycin, Sagamicin, Apramycin, Verdamicin, Xylostasin, Destomycin, Hygromycin, Seldomycin, Umtamicin, Tobramycin, Septinomycin und Fortimicin.

Besonders bevorzugt aus dieser Gruppe sind vor allem Gentamicin, Sisomicin, Etomicin, Amikacin, Streptomycin, Neomycin, Kanamycin, Tobramycin und Fortimicin.

In Frage kommen erfindungsgemäß auch Aminoglykoside, die an ihren Hydroxyl- und/oder Aminogruppen derivatisiert sind. Es sind dies beispielsweise, Derivate gemäß DE—OS 2 712 160 (Aminoglykoside, die an mindestens einem N-Atom einen eine Ether- oder Thioethergruppe aufweisenden Alkyl- oder Acylrest tragen), DE—OS 2 924 659 (Aminoglykoside, die an mindestens einem N-Atom einen Aminohydroxyalkylrest tragen), DE—OS 2 753 769 (1-N-Carbamoyl- und 1-N-Alkoxycarbonyl-Aminoglykoside), DE—OS 2 832 268 (Aminoglykoside, die an mindestens einen N-Atom einen Polyhydroxyalkylrest tragen), DE—OS 2 921 973 (1 - N - Hydroxyalkylaminoalkyloxycarbonyl - Sisomicin), DE—OS 2 928 183 (Sisomicin, am 1-N-Atom mit sekundärem Alkyl substituiert), DE—OS 3 100 739 (am 5-O-Atom durch eine Carbamoylgruppe substituiertes Sisomicin) und DE—OS 3 101 376 (1-Hydroxyalkyl-urethan-Sisomicin) sowie die Pseudodisaccharide gemäß DE—OS 2 730 372.

Als Säurekomponente der erfindungsgemäßen Salze kommen alle selbst nicht antibiotisch wirkenden organischen Carbonsäuren oder Sulfonsäuren sowie auch Halbester von anorganischen Säuren mit aliphatischen Alkoholen in Frage, die mit dem jeweiligen Aminoglykosid(derivat) ein in Wasser schwer lösliches Salz bilden. Beispielhaft seien genannt:

Embonsäure oder Pamoa-Säure (=4,4' - Methylen - bis - [3 - hydroxy - 2 - naphthalincarbonsäure]), 1,5 - Naphthalin - disulfonat oder höhere Fettsäuren, z.B. Dodecansäure, Tetradecansäure, Hexadecansäure, Octadecansäure, Cerotinsäure, Ölsäure, Elaidinsäure, Linolsäure oder β-Hydroxymyristinsäure.

Bevorzugt sind unter diesen Säuren Embonsäure und gegebenenfalls ungesättigte Fettsäuren mit 8—25 C-Atomen, insbesondere 12—18 C-Atomen. In Betracht kommen auch Halbester aus zwei- oder mehrbasigen anorganischen oder organischen Säuren mit aliphatischen Alkoholen wie Monocetylsulfat oder Monocetylmalonat oder auch Ethylsuccinat. Bevorzugt sind Halbester von langkettigen (8—25, vorzugsweise 12—18 C-Atome) Alkoholen.

Bevorzugt werden die erfindungsgemäßen Salze in Kombination mit β-Lactamantibiotika eingesetzt, wobei letztere sowohl in Form der freien Säure als auch in Salzform vorliegen können. Erfindungsgemäß kommen in diesem Zusammenhang alle an sich bekannten β-Lactamantibiotika in Betracht, z.B. Penicilline, Cephalosporine und Oxacepheme. Beispielhaft gennant seien Ampicillin, Propicillin, Oxacillin, Dicloxacillin, Carbenicillin, Azidocillin, Mezlocillin, Azlocillin, Penicillin G, Procain-Penicillin G, Pivampicillin, Amoxicillin, Flucloxacillin, Ticarcillin, Carindacillin, Cielacillin, Epicillin, Cefaloridin, Cefalothin, Cefazolin, Cefamandol, Cefoxitin, Cefuroxim, Cephalexin, Cefradin, Cefaclor, Cefadroxil, Thienamycin und Cefotaxim.

Die schwer löslichen Salze der Aminoglykosidantibiotika können hergestellt werden, indem man wäßrige Lösungen von freien basischen Aminoglykosiden oder von deren neutralen Salzen mit anorganischen oder organischen Säuren zur Reaktion bringt mit wäßrigen Lösungen von Salzen z.B. der höheren Fettsäuren oder der Embonsäure. Die Reaktionstemperatur liegt in der Regel im Bereich zwischen 5° und 95°C, bevorzugt zwischen 50° und 80°C. Der gebildete schwer lösliche Niederschlag wird anschließend abgesaugt, mit Wasser gut nachgewaschen und nach den üblichen Methoden getrocknet.

Als Trägerflüssigkeiten kommen zur Injektion geeignet Öle und Fette in Frage. Injizierbare Öle bzw. Fette sind bei Zimmertemperatur meist flüssige Fette, z.B. Erdnuß-, Mais-, Mandel-, Oliven-, Ricinus oder Sesamöl, oder Fettsäureester, z.B. Ethyloleat oder Isopropylmyristat, die als Lösungsmittel für zu injizierende Stoffe oder zur Herstellung von intravenös zu verabreichenden Fettemulsionen verwendet werden können. Sie müssen in der Bundesrepublik den Vorschriften für "Injectabile" entsprechen, also gemäß den Arzneibuchvorschriften hergestellt werden (vgl. hierzu auch K. Münzel, J. Büchi und O. E. Schultz, Galenisches Praktikum, Wiss. Verlagsges. mbH, Stuttgart (1959), und Arzneibereitung, Ferdinand Enke-Verlag, Stuttgart (1969). Nach der CH—PS 349 750 (v. 4.6.56/15.12.60; C. 1961. 9893) stellt man ein injizierbares F. her, indem man z.B. 500 g Olivenöl mit einem Gemisch von 84 g Glycerin und 5 g Natriumhydroxid 8 Stunden auf 85°C erwärmt. Die obere, Mono- und Diglyceride enthaltende Phase wird abgetrennt, gewaschen und getrocknet, M. M. A. Guerbet (GB—PS 1 81 551 v. 31.8.67, FR—PS v. 18.2.64; C. A. 67. Nr. 120191 (1967)) empfiehlt als injizierbare Fettgrundlage pflanzliche Öle, deren Alkoholyseprodukte oder halogenierte Derivate, und zwar in Kombination mit Polyethylenglykol 400-monopalmitat und einem Tartarsäureester der Monoglyceride des Baumwollsaatöles. Außerdem kommen in Frage: Sojabohnenöl, Miglyol 812®, Viscoleo® und Arlacel A®.

Besonders bevorzugt kommen zur Injektion geeignete Öle bzw. Fette in Frage, z.B. natürliche wie Sesamöl, Erdnußöl, Ricinusöl, Mandelöl, Maiskeimöl oder Olivenöl oder synthetische Triglyceride wie z.B. ein Triglyceridgemisch gesättigter Pflanzenfettsäuren mittlerer Kettenlänge ($C_8$—$C_{12}$), Capryl/Caprinsäure-Triglycerid.

Der Suspensionsformulierung können andere Hilfsstoffe zugegeben werden z.B. Netzmittel wie Lecithin oder Polyoxethylen-Sorbitanmonooleat, Verdickungsmittel wie Aluminium-monostearat, Konservierungsmittel wie Phenole, Benzylalkohol oder Hydroxybenzoesäureester.

Zur Herstellung der Formulierung können die Anteile der Wirk- und Hilfsstoffe in folgendem Rahmen variieren:

Die wirksamen Aminoglykoside können in einem Bereich von 0,5 bis 30 Prozent, vorzugsweise von 1 bis 15 Prozent bezogen auf die Aminoglykosidbase in das Vehikel eingebracht werden.

Der Anteil der Hilfsstoffe kann zwischen 0,05 und 10 Prozent, vorzugsweise 0,2 bis 5 schwanken.

Der Anteil der Penicilline in der Kombinationsformulierung kann zwischen 1 und 40 Prozent, vorzugsweise 3 bis 30 Prozent betragen.

Die Herstellung der spritzfertigen Suspension geschieht in einer in der Pharmazie bekannten Weise unter aspetischen Bedingungen.

Die gewünschten Wirkstoffe werden steril auf eine bestimmte Korngröße hin gefällt oder es wird das sterile Produkt unter aseptischen Bedingungen gemahlen. Die Korngröße sollte dabei zwischen 2 und 60 mcm liegen, vorzugsweise aber zwischen 5 und 30 mcm.

Die gemahlene Substanz wird in das vorher sterilisierte Lösungsmittel eingebracht und unter aseptischen Bedingungen homogenisiert. Die homogene Suspension wird unter sterilen Bedingungen abgefüllt.

Die folgenden Beispiele sollen die Erfindung verdeutlichen jedoch nicht einschränken.

Die im folgenden aufgeführten Formulierungsbeispiele wurden nach obiger Vorschrift unter einem Laminar Flow Zelt hergestellt.

Die Angaben zur Zusammensetzung sind G/V.

# 0 115 001

| Einsatzstoffe | Beispiele | | | | | | | |
|---|---|---|---|---|---|---|---|---|
| | A | B | C | D | E | F | G | H |
| Etomicinsulfat (≙1% Base) | 1,6 g | 1,6 g | | | | | | |
| Natriumsulfit | 0,2 g | | | | | | | |
| Wasser pro injektion | ad 100 ml | | | | | | | |
| 2 %iges Aluminium monostearat Gel mit Sesamöl | | ad 100 ml | | | | ad 100 ml | | |
| Etomicin-Embonat (≙1,0 g Base) | | | 3,46 g | | | 3,46 g | | |
| Ethyloleat | | | ad 100 ml | | | | | |
| Etomicin-Hexadecanat (≙1,0 g Base) | | | | 4,15 g | | | | |
| Etomicin-Dodecanat (≙1,0 g Base) | | | | | 3,49 g | | | |
| Sesamöl | | | | ad 100 ml | ad 100 ml | | ad 100 ml | ad 100 ml |
| Penicillin G Kalium | | | | | | 6,30 g | | |
| Gentamicin-Embonat (≙1,0 g Base) | | | | | | | 3,48 g | |
| Sisomicin-pentaembonat (≙1,0 g Base) | | | | | | | | 3,69 g |

Beispiel I
a) Zusammensetzung

| | |
|---|---|
| Etomicinsulfat gem., steril | 1,77 g |
| Penicillin G Kalium gem., | 1,55 g (≙2,5 Mio E) |
| Miglyol 812® (=synthet. Triglycerid) | ad 100 ml |

b) Herstellung wie oben beschrieben
c) Haltbarkeit der Formulierung

| | Etomicinsulfat best. als Base | Penicillin G Kalium Mio E |
|---|---|---|
| Anfangsgehalt | 0,85% | 2,36% |
| 3 Mon. gelagert bei 20°C | 0,84% | 2,44% |
| 3 Mon. gelagert bei 35°C | 0,86% | 2,37% |
| 6 Mon. gelagert bei 20°C | 0,89% | 2,37% |

5

Beispiel J
a) Zusammensetzung

| | |
|---|---|
| Etomicinsulfat gem., steril | 1,77 g |
| Procain Penicillin G gem., steril | 9,79 g |
| Aluminium-monostearat 2% Gel in Sesamöl | ad 100 ml |

b) Herstellung wie oben beschrieben
c) Haltbarkeit der Formulierung

| | Etomicinsulfat als Base | Procain Penicillin G.K. |
|---|---|---|
| Anfangsgehalt | 0,93% | 10,1% |
| 3 Mon. gelagert bei 20°C | 0,90% | 10,5% |
| 3 Mon. gelagert bei 35°C | 0,85% | 10,25% |
| 6 Mon. gelagert bei 20°C | 1,05% | 9,5% |

Beispiel K
a) Zusammensetzung

| | |
|---|---|
| Sisomicinsulfat | 1,77 g |
| Oxacillin Natrium | 1,50 g |
| Tocopherol | 0,30 g |
| Erdnußöl | ad 100 ml |

b) Herstellung wie oben beschrieben
c) Haltbarkeit der Formulierung

| | Sisomicinsulfat berech. als Base | Oxacillin-Natrium |
|---|---|---|
| Anfangsgehalt | 1,0% | 1,45% |
| nach 1 Mon. Lagerung im Kühlschrank | 0,98% | 1,42% |
| bei 20°C | 1,02% | 1,41% |
| bei 30°C | 1,07% | 1,44% |

Beispiel L
a) Zusammensetzung

| | |
|---|---|
| Etomicinsulfat | 1,77 g |
| Ampicillin Trihydrat | 1,50 g |
| Lecithin | 0,50 g |
| Ethyloleat | ad 100 ml |

b) Herstellung wie oben beschrieben
c) Haltbarkeit der Formulierung

| | Etomicinsulfat als Base | Ampicillin als Säure |
|---|---|---|
| Anfangsgehalt | 0,97% | 1,39% |
| nach 1 Mon. Lagerung im Kühlschrank | 0,98% | 1,32% |
| bei 20°C | 0,99% | 1,33% |
| bei 30°C | 0,95% | 1,22% |

Weitere stabile Formulierungen sind z.B.:

Beispiel M

| | |
|---|---|
| Etomicinsulfat (entsprechend 3,0 g Base) | 5,217 g |
| Procain Penicillin G | 30,000 g |
| Lecithin | 0,1 g |
| Miglyol 812® (synthet. Triglycerid) | ad 100 ml |

Beispiel N

| | |
|---|---|
| Gentamicinsulfat (entsprechend 3,0 g Base) | 5,115 g |
| Penicillin G Kalium | 30,000 g |
| Ethyloleat | ad 100 ml |

Beispiel O

| | |
|---|---|
| Gentamicinembonat (entspricht 2,0 g Base) | 7,000 g |
| Penicillin G Kalium | 1,550 g |
| N,N'-Dibenzylethylendiamin-Salz der Penicillin-G-Säure | 20,000 g |
| Lecithin | 2,000 g |
| Miglyol 812® | ad 100 ml |

Beispiel P

| | |
|---|---|
| Etomicinembonat (≙2,0 g Base) | 6,92 g |
| Etomicinsulfat (≙1,0 g Base) | 1,60 g |
| Procain-Penicillin G | 25,00 g |
| Lecithin | 0,02 g |
| Capryl/Caprinsäure-Triglycerid | ad 100 ml |

Von den Formulierungen wurden in folgender Weise die Blutspiegel des Aminoglykosids bestimmt: Die Formulierungen wurden in entsprechenden Dosierungen bei Beagle-Hunden intramuskulär appliziert. Zu verschiedenen Zeiten nach Behandlung wurden Blutproben entnommen und durch Zentrifugation das Serum gewonnen. Die Bestimmung des Wirkstoffgehaltes im Serum erfolgte mikrobiologisch mit Hilfe des Agardiffusionstestes (Lochtest). Als Nachweiskeim wurde der Stamm Bacillus subtilis ATCC 6633, als Testmedium DST-Agar verwendet. Anhand eines Standards in Puffer wurde der Wirkstoffgehalt in den verschiedenen Serumproben bestimmt. Die Ergebnisse (siehe Tabelle) zeigen, daß nach Applikation der schwerlöslichen Salze therapeutisch relevante Blutspiegel über wesentliche längere Zeit erreicht wurden, als z.B. mit den entsprechenden wasserlöslichen Sulfaten. Infolge der deutlichen Verlängerung der Halbwertszeit bieten diese Formulierungen entscheidende Vorteile für die Therapie.

# 0 115 001

## TABELLE 1
### Serumspiegel Hund/intramuskulär

| Formulierungs-beispiele | Etomicinsalz | Dosis mg/kg | Halbwerts-zeit (h) | Serumkonzentration (γ/ml) | | |
|---|---|---|---|---|---|---|
| | | | | 1 h | 8 h | 12 h |
| B | Sulfat | 4 | 1 | 9,5 | 0,2 | — |
| C | Embonat | 4 | 1,8 | 5 | 0,7 | — |
| | Sulfonat+ Embonat | 4 | 2 | 7,5 | 0,46 | — |
| E | Dodecanat | 4 | 2,5 | 8,8 | 1,3 | 0,36 |
| | | 4 | 2,3 | 7,8 | 0,7 | 0,1 |
| | | 1 | 2 | 1,4 | 0,15 | — |
| D | Hexadecanat | 4 | 3,9 | 4,4 | 1,3 | 0,36 |
| | | 4 | 3,9 | 3,1 | 0,84 | 0,45 |
| | | 1 | 2,4 | 2 | 0,3 | — |

## Patentansprüche

1. Verwendung von wenigstens einem zur Injektion geeigneten Öl, wenigstens einem Aminoglykosid und wenigstens einem Penicillin zur Herstellung von Injektionsformulierungen zur Applikation in der Human- und Veterinärmedizin, ausgenommen Formulierungen zur Infusion in Körperhöhlen.

2. Verwendung gemäß Anspruch 1 von 0,5 bis 30 Gew.-% wenigstens eines Aminoglykosids, 1 bis 40 Gew.-% wenigstens eines Penicillins sowie gegebenenfalls 0,05 bis 10 Gew.-% weiterer Formulierungshilfsmittel zur Herstellung von Injektionsformulierungen zur Applikation in der Human- und Veterinärmedizin ausgenommen zur Infusion in Körperhöhlen.

3. Verwendung gemäß Anspruch 1 von Wirkstoffpaaren

Etomicin-Sulfat/Penicillin G-Kalium
Etomicin-Sulfat/Procain Penicillin G
Siromicin-Sulfat/Oxacillin-Natrium
Etomicin-Sulfat/Ampicillin Trihydrat
Gentamicin-Sulfat/Penicillin G-Kalium
Gentamicin-Embonat/Penicillin G-Kalium
Etomicin-Embonat/Procain-Penicillin G

und einem zur Injektion geeigneten Öl zur Herstellung von Injektionsformulierungen zur Applikation in der Human- und Veterinärmedizin ausgenommen zur Infusion in Körperhöhlen.

## Revendications

1. Utilisation d'au moins une huile convenant pour l'injection, d'au moins un aminoglycoside et d'au moins une pénicilline pour la préparation de formulations d'injection en vue de l'application en médecine humaine et vétérinaire, à l'exclusion de formulations pour l'infusion dans des cavités corporelles.

2. Utilisation selon la revendication 1 de 0,5 à 30% en poids d'au moins un aminoglycoside, de 1 à 40% en poids d'au moins une pénicilline de même qu'éventuellement de 0,05 à 10% en poids d'autres auxiliaires de formulation pour la préparation de formulations d'injection en vue de l'application en médecine humaine et vétérinaire, à l'exclusion de celles pour l'application dans des cavités corporelles.

3. Utilisation selon la revendication 1 de paires de matières actives:

Sulfate d'étomicine/Pénicilline G-potassium
Sulfate d'étomicine/Procaïne-Pénicilline G
Sulfate de siromicin/Oxacilline-sodium
Sulfate d'étomicine/Trihydrate d'ampicilline

**0 115 001**

Sulfate de gentamicine/Pénicilline G-potassium
Embonate de gentamicine/Pénicilline G-potassium
Embonate d'étomicine/Procaïne-Pénicilline G

et d'une huile convenant pour l'injection en vue de la préparation de formulations d'injection pour l'application en médecine humaine et vétérinaire, à l'exclusion de celles pour l'infusion dans des cavités corporelles.

**Claims**

1. Use of at least one oil suitable for injection, at least one aminoglycoside and at least one penicillin for the preparation of formulations for injection for administration in human and veterinary medicine, except formulations for infusion into body cavities.

2. Use according to Claim 1 of 0.5 to 30% by weight of at least one aminoglycoside, 1 to 40% by weight of at least one penicillin and optionally 0.05 to 10% by weight of other formulating auxiliaries for the preparation of formulations for injection for administration in human and veterinary medicine, except for infusion into body cavities.

3. Use according to Claim 1 of the following pairs of active compounds

Etomicin sulphate/Penicillin G Potassium
Etomicin sulphate/Procaine penicillin G
Siromicin (sic) sulphate/Oxacillin sodium
Etomicin sulphate/Ampicillin trihydrate
Gentamicin sulphate/Penicillin G potassium
Gentamicin embonate/Penicillin G potassium
Etomicin embonate/Procaine penicillin G

and of an oil suitable for injection for the preparation of formulations for injection for administration in human and veterinary medicine, except for infusion into body cavities.